# EUROPEAN PATENT APPLICATION

(11) **EP 4 029 527 A1**
(43) Date of publication of application: **20.07.2022**
(21) Application number: 21151289.2
(22) Date of filing: 13.01.2021
(51) Int. Cl.: A61L 2/10, A61L 2/24, E05B 1/00, E03D 9/00, A47K 13/00

(54) **SETTING A STATE OF UV RADIATION SOURCES IN DEPENDENCE ON INPUT FROM A DETECTOR ARRANGEMENT**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); NIESSEN, Eduard Matheus Johannes, 5656 AE Eindhoven (NL); Martens, Peter, 5656 AE Eindhoven (NL)
(74) Representative: Philips Intellectual Property & Standards

(57) **Abstract**

A system (1) comprises a radiation body (10), a radiation arrangement (20) configured to provide radiation at least comprising UV radiation (25) to the radiation body (10), a detector arrangement (30) configured to detect an internal radiation intensity of radiation in the radiation body (10) at at least one detector position, and a controller arrangement (40) for applying a controller algorithm configured to set a state of UV radiation sources (21, 22) of the radiation arrangement (20) in dependence on input from the detector arrangement (30). In particular, when the input from the detector arrangement (30) represents a change of the internal radiation intensity that is qualified as a disturbance incident at the at least one detector position, actions of identifying at least one involved UV radiation source (21, 22) and setting an adapted state of the at least one involved UV radiation source (21, 22) are performed.

## Description

### FIELD OF THE INVENTION

The invention relates to a system comprising a radiation body comprising a radiation exit window, wherein the radiation body is configured to receive radiation that at least comprises UV radiation, and to radiate at least part of the radiation to the exterior of the radiation body via the radiation exit window; a radiation arrangement configured to provide the radiation, comprising at least two UV radiation sources configured to emit UV radiation; a detector arrangement configured to detect an internal radiation intensity of radiation in the radiation body at at least one detector position; and a controller arrangement, functionally coupled to the radiation arrangement and the detector arrangement.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element, an optical sensor and a control system. The waveguide element comprises a radiation exit window, wherein the waveguide element is i) configured to receive radiation, wherein the radiation at least comprises UV radiation, ii) configured to radiate at least part of the radiation to the exterior of the waveguide element via the radiation exit window, and iii) configured to internally reflect part of the radiation at the radiation exit window. The optical sensor is configured to sense an internal reflection intensity of the internally reflected radiation. The control system is functionally coupled to the optical sensor and is configured to reduce the intensity of the radiation as function of reaching a predetermined first threshold of a reduction of the internal reflection intensity over time.

WO 2018/215272 A1 teaches that the UV radiation is used for killing microorganisms as may be present on the radiation exit window, or for rendering the microorganisms inactive or unable to reproduce. As a practical example of microorganisms, bacteria are mentioned. The disinfecting effect of using UV radiation is mainly governed by a total dose of the UV radiation. In the context of using UV radiation, it may be necessary to take specific measures when higher organisms, including human beings, may be in a position of receiving the UV radiation, which is especially the case if it is possible for the higher organisms to physically contact radiation emitting surfaces.

During operation of the known system, radiation is coupled out of the waveguide element through the radiation exit window if some object touches the window. This outcoupling means that less radiation will stay inside the waveguide element and is also referred to as frustration of the (total) internal reflection. Therefore, by using the optical sensor to monitor an internal reflection intensity of the internally reflected radiation, it is possible to detect a situation of some object touching the window, especially some object that is considerably larger than microorganisms, such as a human hand or finger. When a considerable step is observed in the signal provided by the optical sensor, it is assumed that this means that a relatively large object contacts the window. In order to ensure safety in such a situation, it may be determined that the radiation needs to be shut off, at least temporarily.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications of the invention that is the subject of WO 2018/215272 A1, it is important that external surfaces are kept in a disinfected state while situations in which the UV radiation used in the process might cause harm to higher organisms such as human beings need to be avoided.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. However, the same principle of outcoupling the radiation at contact positions would cause potentially unsafe exposure of human beings touching the waveguide element to UV radiation if it was not for the measure of detecting the touch and shutting off the radiation. In the context of the present invention, it is acknowledged that there is a need for developing useful ways of actually putting this measure to practice.

### SUMMARY OF THE INVENTION

It is an object of the invention to design a system comprising a radiation body and a radiation arrangement in such a way that a proper balance is realized between effectively obtaining disinfection results at the position of a radiation exit window of the radiation body on the one hand and protecting human beings and other higher organisms from harmful exposure to UV radiation provided by the at least two UV radiation sources of the radiation arrangement on the other hand. In WO 2018/215272 A1, an option that is suggested in respect of reducing the intensity of UV radiation when contact with a higher organism is detected involves locally switching off the UV radiation or locally reducing the intensity of the UV radiation, and the invention aims to provide a practical way of implementing this option. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation, and to radiate at least part of the radiation to the exterior of the radiation body via the radiation exit window; a radiation arrangement configured to provide the radiation, comprising at least two UV radiation sources configured to emit UV radiation; a detector arrangement configured to detect an internal radiation intensity of radiation in the radiation body at at least one detector position; and a controller arrangement, functionally coupled to the radiation arrangement and the detector arrangement, configured to set a normal state of the UV radiation sources as a default, in which state the UV radiation sources provide the UV radiation with an intensity at an operational level, and configured to receive input from the detector arrangement and to apply a controller algorithm that involves i) determining at least one safety test value that is representative of a parameter of the internal radiation intensity detected by the detector arrangement at the at least one detector position, ii) assessing whether the at least one safety test value is inside or outside of a safety range of values associated with the at least one detector position, iii) after occurrence of a disturbance incident in which the at least one safety test value shifts from inside the safety reference range of values to outside of the safety reference range of values, identifying at least one involved UV radiation source of which a change of contribution to an internal radiation intensity of UV radiation is demonstrated through occurrence of the disturbance incident, and iv) setting an adapted state of the at least one involved UV radiation source, in which state the at least one involved UV radiation source provides the UV radiation with an intensity at a level that is reduced relative to the operational level.

It follows from the foregoing that the system according to the invention comprises i) a radiation body comprising a radiation exit window, wherein the radiation body is configured to receive radiation that at least comprises UV radiation, and to radiate at least part of the radiation to the exterior of the radiation body via the radiation exit window, ii) a radiation arrangement configured to provide the radiation, comprising at least two UV radiation sources configured to emit UV radiation, iii) a detector arrangement configured to detect an internal radiation intensity of radiation in the radiation body at at least one detector position, and iv) a controller arrangement, functionally coupled to the radiation arrangement and the detector arrangement. For the sake of clarity, it is noted that the indication that the detector arrangement is configured to detect an internal radiation intensity of radiation in the radiation body at at least one detector position may imply in practice that the detector arrangement is capable of obtaining data for calculating and outputting an arbitrary parameter related to the internal radiation intensity at the at least one detector position, wherein it is not necessary that the detector arrangement is capable of determining the actual value of the internal radiation intensity at the at least one detector position.

The controller arrangement of the system according to the invention is configured to set a normal state of the UV radiation sources as a default. Further, the controller arrangement is configured to process input received from the detector arrangement in such a way that occurrence of a disturbance incident can be determined in respect of at least one detector position, and at least one involved UV radiation source of which a change of contribution to an internal radiation intensity of UV radiation is demonstrated through occurrence of the disturbance incident can be identified. This allows the controller arrangement to set an adapted state of the at least one involved UV radiation source while maintaining the normal state of any UV radiation source other than the at least one involved UV radiation source. In this way, assuming that occurrence of a disturbance incident is indicative of the radiation exit window being contacted by a relatively large object such as a human hand or finger, the controller arrangement is configured to enable disinfection of the radiation exit window as a default, and to implement a safety measure of reducing the intensity at which UV radiation is provided whenever appropriate. The latter is typically done at a local level, namely at the level of the at least one UV radiation source that is identified as being involved on the basis of a disturbance incident found at a detector position. As a result, the general disinfection functionality of the system is only limited to a minimum extent while safety can be guaranteed at all times. In this respect, it is noted that the system may be designed in any appropriate way to realize/enable restoration of the default state of the at least one involved UV radiation source in any possible suitable fashion after the adapted state of the at least one involved UV radiation source has been set, and that the controller arrangement may particularly be configured to maintain the adapted state of the at least one involved UV radiation source only during a limited amount of time. The reduced level of the intensity of the UV radiation provided by the at least one involved UV radiation source in the adapted state may be a zero level, although this is not necessary in the context of the invention.

Occurrence of a disturbance incident is determined when it appears that a safety test value that is representative of a parameter of the internal radiation intensity detected by the detector arrangement at the at least one detector position is outside of a safety reference range of values associated with the at least one detector position. According to a first practical option, the safety test value may be representative of a value of the internal radiation intensity of the radiation detected by the detector arrangement. According to a second practical option, the safety test value may be representative of a derivate of the value of the internal radiation intensity of the radiation detected by the detector arrangement, particularly a value of change of the internal radiation intensity in predetermined direction (i.e. to a higher value or a lower value). In respect of the first practical option, the controller arrangement may be configured to assess whether or not a value representing the value of the internal radiation intensity is above or below a predetermined threshold. In respect of the second practical option, the controller arrangement may be configured to calculate a value representing a difference between values of the internal radiation intensity of subsequent detection actions and to assess whether or not the value is larger than a predetermined threshold and/or to calculate a value representing a rate of change of the internal radiation intensity in predetermined direction and to assess whether or not the value is larger than a predetermined threshold. The invention covers various options which may be at the basis of a change of the internal radiation intensity and which are related to the constitution of the system, particularly the features of the radiation body, including the option of such a change resulting from an increase of radiation exiting the radiation body, the option of such a change resulting from an increase of radiation being reflected into the radiation body, and the option of such a change resulting from a combination of a change of exiting radiation and a change of incoming radiation.

According to a further insight of the invention, an advantageous side effect of determining at least one safety test value that is representative of a value of the internal radiation intensity of the radiation detected by the detector arrangement is that it is possible to detect failure of the UV radiation sources. This is based on the assumption that failure of one or more UV radiation sources involves a different default value of the safety test value in case the detector arrangement is configured to detect an internal radiation intensity of the UV radiation. In view thereof, it may be so that the controller arrangement is configured to apply a failure detection algorithm that involves assessing whether or not a time trend of the safety test value in periods of default operation of the UV radiation sources shows a structural change. If such a structural change is found, indeed, this may be taken as an indication of failure of one or more UV radiation sources.

The number of detector positions of the detector arrangement can be chosen freely. In this respect, it may be useful to relate the number of detector positions to the size of the radiation exit window and/or to the number of UV radiation sources. In respect of the latter option, it is explicitly noted that the number of detector positions may or may not be the same as the number of UV radiation sources. In any case, in an embodiment of the system in which the detector arrangement is configured to detect an internal radiation intensity of radiation in the radiation body at at least two different detector positions, there is a safety range of values for each of the detector positions, and the steps of determining at least one safety test value and assessing whether the at least one safety test value is inside or outside of a safety range of values is performed per detector position. In this respect, it is noted that the invention covers both possibilities of the safety range of values being the same and different for two different detector positions. For the sake of completeness, it is noted that in the embodiment of the system as mentioned, i.e. the embodiment in which detection of the internal radiation intensity takes place at more than one detector position, it is possible that the input received from the detector arrangement indicates occurrence of a disturbance incident at more than one detector position. Such input may be representative of the radiation exit window being contacted by an object that is of such a size that the effect thereof on the internal radiation intensity can be found at more than one detector position and/or of the radiation exit window being contacted by a number of objects at the same time.

A notable aspect of the invention is that when occurrence of a disturbance incident is found at a detector position, at least one involved UV radiation source is identified, an involved UV radiation source being a UV radiation source of which a change of contribution to an internal radiation intensity of UV radiation is demonstrated through occurrence of the disturbance incident.

According to a first practical option, the controller algorithm involves identifying the at least one involved UV radiation source on the basis of a predetermined relation between the UV radiation sources and the at least one detector position. This implies that it may be so that a predetermined radiation subset including at least one UV radiation source may be assigned to each of the detector positions. Each of the UV radiation sources is included in at least one of the radiation subsets. When occurrence of a disturbance incident is found in respect of a detector position, the at least one UV radiation source of the radiation subset related to the detector position is denoted as being at least one involved UV radiation source and is put to the adapted state.

According to a second practical option, the controller algorithm involves identifying the at least one involved UV radiation source by performing an identification procedure in which respective radiation subsets are successively put to the normal state while all of the other radiation subsets (which may be one or more other radiation subsets) are kept in an inactive state so that it may be determined in respect of which of the radiation subsets conditions of normal internal radiation intensity are found and in respect of which of the radiation subsets conditions of the disturbance incident are found. In particular, such an identification procedure involves the following: i) controlling the radiation arrangement to realize different UV radiation situations by successively putting different radiation subsets including at least one UV radiation source to the normal state while keeping all of the other UV radiation subsets in an inactive state, ii) controlling the detector arrangement to detect the internal radiation intensity of the UV radiation at the at least one detector position in respect of each of the different UV radiation situations, iii) determining an identification test value that is representative of a value of the internal radiation intensity detected by the detector arrangement at the at least one detector position in respect of each of the different UV radiation situations, and iv) assessing whether the identification test value is inside or outside of an identification range of values associated with the at least one detector position in respect of each of the different UV radiation situations. The identification range of values associated with the at least one detector position in respect of each of the different radiation situations may be fixed, but it is also possible that the controller arrangement is configured to enable adjustment of the identification range of values. The latter may help to compensate for performance differences of the UV radiation sources over time, for example, or for performance differences due to replacement of the UV radiation sources, and may thereby contribute to accuracy of the identification procedure.

In an advantageous embodiment of the system according to the invention, the controller arrangement is configured to enable adjustment of the safety reference range of values associated with the at least one detector position. In this respect, according to one feasible option, the controller arrangement is configured to perform an action of adjusting the safety reference range of values on the basis of a preceding value of the internal radiation intensity detected at the at least one detector position during a period directly prior to occurrence of a disturbance incident so as to include the preceding value and a predetermined tolerance window. In that way, it can be guaranteed that the safety reference range of values is representative of an actual constitution of the system at the very time of the disturbance incident. Alternatively, according to another alternative option, the controller arrangement is configured to perform an action of adjusting the safety reference range of values on the basis of a value of the internal radiation intensity detected at the at least one detector position during the largest (consecutive) part of a 24 hour time period so as to include that value and a predetermined tolerance window. This option may particularly be useful in situations of the system being applied in the context of public buildings, public transport vehicles or the like which are normally accessed by many people during a defined part of the day only and by considerably less people or no one at all during the remainder of the day. According to yet another feasible option, the controller arrangement is configured to apply a calibration algorithm that involves determining a calibration value of the internal radiation intensity in respect of the at least one detector position during calibration periods at predetermined calibration intervals, and that involves adjusting the safety reference range of values on the basis of the calibration value of the internal radiation intensity so as to include the calibration value and a predetermined tolerance window. The predetermined calibration intervals may be of equal duration and may be 24-hour intervals, for example, and, depending on the practical application of a system, the time at which the calibration algorithm is executed is preferably set to be a time at which the chance of the radiation exit window getting contacted by a higher organism such as a human being is minimal to zero. In a large number of feasible applications of the system, this may imply that a time such as 3 am may be appropriate for executing the calibration algorithm, for example.

Another or additional way of adjusting the safety reference range of values associated with the at least one detector position that is covered by the invention involves having a user interface for receiving user input to that end. Further, it is to be noted that the invention also relates to an option of the safety reference range of values associated with the at least one detector position being a fixed system setting. In any case, information representing reference ranges and/or reference rates can be stored in a database or the like, and the controller algorithm can include a step of retrieving such information from the database or the like. Although it is advantageous to have automatic calibration, the invention also covers an option of the controller arrangement being configured to apply a calibration algorithm that involves determining a calibration value of the internal radiation intensity in respect of the at least one detector position during calibration periods initiated on the basis of user input.

In an embodiment of the system according to the invention, the controller algorithm involves maintaining the normal state of the at least one involved UV radiation source during a delay period of predetermined duration directly after occurrence of a disturbance incident, and setting the adapted state of the at least one involved UV radiation source directly after expiry of the delay period only when there is no occurrence during the delay period of a restoration incident involving a reversed shift of the at least one safety test value. In this way, a reaction of the system is avoided if the radiation exit window is only shortly touched and there is actually no health risk.

It is suggested earlier that the number of detector positions of the detector arrangement may be chosen freely. The detector arrangement may comprise any suitable number of detectors for realizing the functionality of the detector arrangement as envisaged, wherein the invention includes the possibility of having no more than a single detector in the detector arrangement. The number of UV radiation sources of the radiation arrangement may also be chosen freely, wherein a minimum of two UV radiation sources is applicable. Depending on the constitution of the radiation body and the size of the radiation exit window, it may be advantageous if the at least two UV radiation sources are arranged in the radiation body at a distance from each other and/or if the detector arrangement comprises at least two detectors arranged in the radiation body at a distance from each other. Also, it is possible to have more than one detector at one detector position, or nearly the same detector position, wherein it may be practical if the detectors are configured and arranged to detect radiation from different/opposite directions. Likewise, it is possible to have more than one UV radiation source at a certain position, wherein it may be practical if the UV radiation sources are configured and arranged to emit UV radiation in different/opposite directions. It may be practical if the radiation body comprises a slab of material, wherein the at least two UV radiation sources and/or the at least one detector is/are embedded in the slab of material. The material of the slab of material may be silicone, for example.

The UV radiation sources may be of any suitable type, and may be LEDs, particularly UV-C LEDs, for example. In such a case, the detector arrangement may comprise the same LED, for example, in view of the fact that it is possible to use a LED as a radiation detector, wherein it is practical if the controller arrangement is configured to put the LED to a detector state instead of a radiation source state from time to time. For example, the LED could be driven to emit radiation during a major percentage such as 90% or 95% of the time and be turned off during the remainder of the time in order to function as detector. In this respect, it is furthermore possible that the controller arrangement is configured to set the state of LEDs from one group of LEDs to be different from the state of LEDs from another group of LEDs at any given moment. Using LEDs both as radiation source and detector offers a possibility to reduce the amount of components needed and to improve accuracy of determining locations of touch on the radiation exit window and identifying the involved UV radiation sources.

In the context of the invention, it is possible that the detector arrangement is configured to detect an internal radiation intensity of the UV radiation, as suggested earlier. Another option is that the system comprises another type of radiation source besides the UV radiation sources, namely at least one radiation source configured to provide radiation of distinctly longer wavelengths, such as visible light or infrared radiation, and that the detector arrangement is configured to at least detect the internal radiation intensity of the radiation of the distinctly longer wavelengths. In such a case, when the radiation exit window is touched, the at least one involved UV radiation source can be identified from the way in which the touch influences the internal radiation intensity of the radiation of the distinctly longer wavelengths. In view of the fact that absorption of radiation of longer wavelengths is typically significantly lower, the steps of checking whether or not a disturbance incident occurs at the at least one detector position and identifying the at least one involved UV radiation source can be performed with high accuracy, while the number of additional radiation sources and detectors can be kept limited.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

The above-described and other aspects of the invention will be apparent from and elucidated with reference to the following detailed description of practical embodiments of a system comprising i) a radiation body comprising a radiation exit window, wherein the radiation body is configured to receive radiation that at least comprises UV radiation, and to radiate at least part of the radiation to the exterior of the radiation body via the radiation exit window, ii) a radiation arrangement configured to provide the radiation, comprising at least two UV radiation sources configured to emit UV radiation, iii) a detector arrangement configured to detect an internal radiation intensity of radiation in the radiation body at at least one detector position, and iv) a controller arrangement, functionally coupled to the radiation arrangement and the detector arrangement.

### BRIEF DESCRIPTION OF THE DRAWINGS

The invention will now be explained in greater detail with reference to the figures, in which equal or similar parts are indicated by the same reference signs, and in which:
Fig. 1 diagrammatically shows a portion of a system according to a first embodiment of the invention,
Fig. 2 diagrammatically shows a portion of a system according to a second embodiment of the invention,
Fig. 3 diagrammatically shows a system according to a third embodiment of the invention, and
Fig. 4 diagrammatically shows an object that can be equipped with one or more systems according to the invention.

### DETAILED DESCRIPTION OF THE EMBODIMENTS

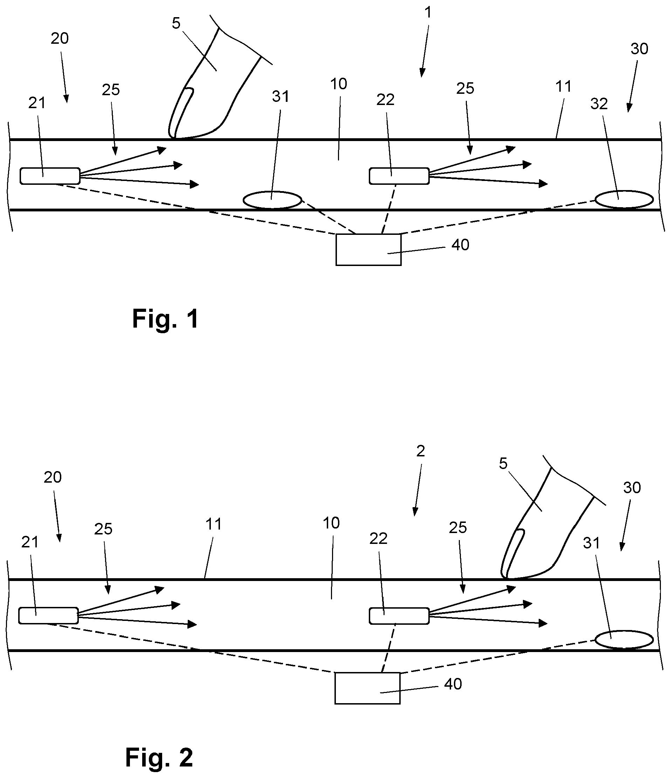

Fig. 1 diagrammatically shows a portion of a system 1 according to first embodiment of the invention.

The system 1 comprises a radiation body 10, a radiation arrangement 20 comprising a number of UV radiation sources of which two UV radiation sources 21, 22 are shown in Fig. 1, a detector arrangement 30 comprising a number of UV radiation detectors of which two UV radiation detectors 31, 32 are shown in Fig. 1, and a controller arrangement 40.

The radiation body 10 comprises a radiation exit window 11. Fig. 1 diagrammatically shows a side view of the radiation body 10 and illustrates the practical options of the radiation body 10 comprising a slab of material and the radiation exit window 11 being of generally flat appearance.

The UV radiation sources 21, 22 are configured to emit UV radiation such as UV-C radiation, in one or more directions, and may be UV-C LEDs, for example. In Fig. 1, UV radiation emitted by the UV radiation sources 21, 22 is depicted by means of a number of arrows 25. Further, Fig. 1 illustrates the practical option of the UV radiation sources 21, 22 being embedded in the slab of material of the radiation body 10, wherein the slab of material is transparent to the UV radiation 25 provided by the UV radiation sources 21, 22. Any appropriate number of UV radiation sources 21, 22 can be chosen in the context of the invention, wherein it is possible to have an arrangement of the UV radiation sources 21, 22 in which the UV radiation sources 21, 22 are positioned to radiate UV radiation 25 to respective areas of the radiation exit window 11 of the radiation body 10, whether or not with overlap. The UV radiation sources 21, 22 can be arranged in any appropriate pattern, including a pattern in which the UV radiation sources 21, 22 are equally distributed throughout the slab of material of the radiation body 10, in an imaginary plane extending parallel to the radiation exit window 11, in a row or in a 2D array. In any case, it is practical if the UV radiation sources 21, 22 are arranged at a distance from each other.

The UV radiation detectors 31, 32 are configured to detect an internal radiation intensity of the UV radiation 25 in the radiation body 10 at respective detector positions. Fig. 1 illustrates the practical option of the UV radiation detectors 31, 32 being embedded in the slab of material of the radiation body 10. Any appropriate number of UV radiation detectors 31, 32 can be chosen in the context of the invention, and may or may not be the same as the number of UV radiation sources 21, 22. The UV radiation detectors 31, 32 can be arranged in any appropriate pattern, including a pattern in which the UV radiation detectors 31, 32 are equally distributed throughout the slab of material of the radiation body 10, in an imaginary plane extending parallel to the radiation exit window 11, in a row or in a 2D array. In any case, it is practical if the UV radiation detectors 31, 32 are arranged at a distance from each other.

The controller arrangement 40 is functionally coupled to the radiation arrangement 20 and the detector arrangement 30 and is configured to control operation of the system 1. Data communication between the controller arrangement 40 and each of the UV radiation sources 21, 22 and the UV radiation detectors 31, 32 is diagrammatically indicated through dashed lines in Fig. 1. The invention covers both an option of the system 1 comprising a communication arrangement configured to enable the data communication to take place in a wired fashion and an option of the system 1 comprising a communication arrangement configured to enable the data communication to take place in a wireless fashion, and also covers an option of the system 1 comprising a communication arrangement configured to enable the data communication to take place partly in a wired fashion and partly in a wireless fashion. The controller arrangement 40 may comprise at least one unit that is positioned outside of the slab of material of the radiation body 10, as illustrated in Fig. 1. Additionally or alternatively, it is possible that the controller arrangement 40 comprises at least one unit that is embedded in the slab of material of the radiation body 10.

The radiation arrangement 20, the detector arrangement 30 and the controller arrangement 40 may be powered in any suitable way and may be electrically coupled to the mains or to a battery, for example.

A general explanation of how the system 1 works is now provided. First, it is to be noted that most materials have a high absorption for radiation in the UV-C wavelength ranges. For example, silicone materials typically absorb over 20% of the radiation, per cm travelled. As a result, after 10 cm, only (1-0.2)¹⁰ = about 10% of the radiation is left, and after 20 cm, this has reduced further to only about 1%. If it is assumed that the material of the slab of material of the radiation body 10 is silicone, that the UV radiation sources 21, 22 are arranged in a row, at a distance of 10 cm from each other, and that the UV radiation detectors 31, 32 are also arranged in a row, at a distance of 10 cm from each other, at intermediate positions between the UV radiation sources 21, 22 as seen in the direction in which the row extends, it is found that roughly speaking, the internal radiation intensity at each of the detector positions is mainly determined by the UV radiation 25 of the nearest UV radiation source 21, 22 in view of the fact that a percentage of the UV radiation 25 of the nearest UV radiation source 21, 22 that is left at a detector position is about 33%, while a percentage of the UV radiation 25 of the second-nearest UV radiation source 21, 22 that is left at the same detector position is only about 3.5%.

When the radiation exit window 11 of the radiation body 10 is touched by an object 5 such as a person's finger, as diagrammatically shown in Fig. 1, this involves a local change of the internal radiation intensity of the UV radiation 25. The fact is that depending on the features of the radiation body 10, phenomena such as reflection and/or scattering of the UV radiation 25 at the position of the radiation exit window 11 are influenced and/or an extent to which UV radiation 25 is coupled out of the radiation body 10 through the radiation exit window 11 changes.

The main purpose of having the radiation arrangement 20 in the system 1 is keeping an exterior side of the radiation exit window 11 in a disinfected state. When bacteria or viruses end up on the exterior side of the radiation exit window 11, the bacteria or viruses are killed or at least rendered inactive under the influence of UV radiation 25 exiting the radiation body 10 through the radiation exit window 11 at the position of the bacteria or viruses. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or animals/pets.

The controller arrangement 40 is configured to control the radiation arrangement 20 in dependence of input provided by the detector arrangement 30, especially when it comes to setting the intensity of the UV radiation 25 emitted by the respective UV radiation sources 21, 22. The fact is that for safety reasons, it is desired to reduce the intensity of the UV radiation 25, probably to zero, in a situation of a person or animal/pet touching the radiation exit window 11, at the position of the touch and probably a surrounding area of minimal size only, so that the disinfecting functionality is maintained at the other positions and not unnecessarily reduced/removed as well. In view thereof, the controller arrangement 40 is configured to set a normal state of the UV radiation sources 21, 22 in which the UV radiation sources 21, 22 provide the UV radiation 25 with an intensity at an operational level as a default, and to set an adapted state of at least one of the UV radiation sources 21, 22 in which the at least one of the UV radiation sources 21, 22 provides the UV radiation 25 with an intensity at a level that is reduced relative to the operational level after occurrence of a disturbance incident involving a change of the internal radiation intensity detected by the detector arrangement 30 at at least one detector position, in case the at least one of the UV radiation sources 21, 22 is identified as being involved on the basis of the disturbance incident. In this respect, it is noted that occurrence of a disturbance incident is determined when a safety test value is outside of a safety reference range of values, wherein the safety test value and the safety reference range of values may relate to a value of the internal radiation intensity of the UV radiation 25 detected by the detector arrangement 30 or a value of change in predetermined direction of the internal radiation intensity of the radiation detected by the detector arrangement.

Determining which at least one of the UV radiation sources 21, 22 is identified as involved UV radiation source 21, 22 when occurrence of a disturbance incident is found at a detector position can be done in several ways. According to one option, at least one UV radiation source 21, 22 is identified as involved UV radiation source 21, 22 on the basis of a predetermined relation between the detector positions on the one hand and the UV radiation sources 21, 22 on the other hand. This means that every time a disturbance incident occurs at a detector position, one or more UV radiation sources 21, 22 are automatically identified as being involved UV radiation sources 21, 22. According to another option, after occurrence of a disturbance incident at a detector position, the UV radiation sources 21, 22 are shut off and respective radiation subsets including at least one UV radiation source 21, 22 are successively put to the normal state in order to assess on the basis of input from the detector arrangement 30 which at least one of the radiation subsets can be linked to the disturbance incident. This does not need to take long, and the time that a UV radiation source 21, 22 is put to the normal state may be as short as 1 millisecond, for example. After the at least one involved radiation subset has been identified, the UV radiation sources 21, 22 are powered again, wherein the at least one UV radiation source 21, 22 of the at least one involved radiation subset is put to the adapted state.

In order to ensure proper functioning of the system 1, the safety range of values is preferably chosen such that the presence of relatively small objects on the radiation exit window 11, particularly objects having dimensions of tens to hundreds micrometer or even smaller dimensions, such as tiny droplets and/or grease/dirt containing bacteria and/or viruses, cannot cause the controller arrangement 40 to establish occurrence of a disturbance incident, so that the system 1 only reacts in a situation of relatively large objects 5 being present on the radiation exit window 11. In a sophisticated embodiment of the system 1, the controller arrangement 40 is configured to enable adjustment of the safety reference range of values of the internal radiation intensity associated with the one or more detector positions. This may be done on the basis of detection results from a period directly prior to occurrence of a disturbance incident at a detector position or by regularly performing a calibration action at appropriate times. In any case, the invention provides an uncomplicated and cost-effective way of combining the disinfection goal with detection and localization of a possible presence of a higher organism, wherein only the at least one UV radiation source 21, 22 of which UV radiation 25 may reach the higher mechanism in a harmful dosage is put to an adapted state. As a result, the time that the UV radiation sources 21, 22 of the radiation arrangement are operated in a default normal state is maximized and the risk of harmful exposure of a higher organism to the UV radiation 25 is minimized.

Fig. 2 diagrammatically shows a portion of a system 2 according to second embodiment of the invention. As suggested in the foregoing, the number of UV radiation detectors 31, 32 may or may not be the same as the number of UV radiation sources 21, 22. In the system 2 according to the second embodiment of the invention, the number of UV radiation detectors 31, 32 is smaller than the number of UV radiation sources 21, 22. In this respect, it is noted that Fig. 2 shows two UV radiation sources 21, 22 and one UV radiation sensor 31. The UV radiation detector 31 is positioned relative to the UV radiation sources 21, 22 in such a way that the UV radiation source 22 that is closest to the UV radiation detector 31 contributes a relatively large percentage to the internal radiation intensity that is normally detected at the detector position and the UV radiation source 21 that is more remote from the UV radiation detector 31 contributes a relatively small percentage to the internal radiation intensity that is normally detected at the detector position. For example, the relatively large percentage may be a percentage of 90% or more, and the relatively small percentage may be a percentage of 10% or less. In such a configuration, it may be determined that a relatively large object 5 is present on the radiation exit window 11 at a position close to the UV radiation source 22 that is closest to the UV radiation detector 31 when the percentage of change of the internal radiation intensity is well above 10%.

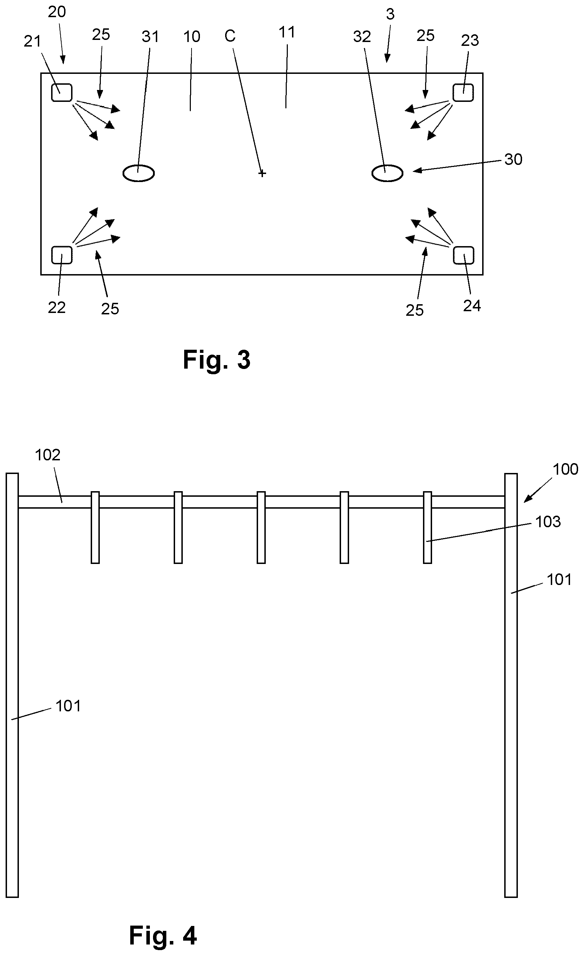

Improved accuracy of determining if a large object 5 is present on the radiation exit window 11 and if so, localizing the object 5, i.e. improved accuracy of determining which part of the radiation arrangement 20 can be operated according to default and which part of the radiation arrangement 20 needs to be operated in an adapted fashion at any given time, can be realized by having at least two detector positions. In this respect, Fig. 3 diagrammatically shows a system 3 according to third embodiment of the invention, which system 3 comprises a radiation body 10 including a rectangular and generally flat slab of material, four UV radiation sources 21, 22, 23, 24 embedded in the slab of material, and two UV radiation detectors 31, 32 embedded in the slab of material. Fig. 3 diagrammatically shows a top view of the radiation body 10 and the embedded UV radiation sources 21, 22, 23, 24 and UV radiation detectors 31, 32. For the sake of clarity, the controller arrangement 40 that is comprised by the system 3 is not shown in Fig. 3.

In the system 3 as shown, the four UV radiation sources 21, 22, 23, 24 are located at the four corner positions of the slab of material of the radiation body 10, and the two UV radiation detectors 31, 32 are located at positions which are between the short sides of the slab of material and a central position C in the slab of material, and which are central between the long sides of the slab of material, like the central position C. The UV radiation sources 21, 22, 23, 24 are configured to emit UV radiation 25 in a direction towards the central position C, and the UV radiation detectors 31, 32 are configured to detect an internal radiation intensity of the UV radiation 25 in the radiation body 10 at all of their sides. In this configuration, in a situation in which the radiation exit window 11 of the radiation body 10 is touched by an object 5 at a central position, a similar change of the internal radiation intensity at each of the two detector positions is caused. On the basis thereof, it may or may not be determined that all of the UV radiation sources 21, 22, 23, 24 should be operated in an adapted fashion, depending on whether or not the level of the UV radiation 25 is considered as harmful to a higher organism at such a central position. In a situation in which the radiation exit window 11 of the radiation body 10 is touched by an object 5 at a position that is more to a short side of the radiation exit window 11, this causes a notable change of the internal radiation intensity at the detector position closest to that position and no or only an insignificant change of the internal radiation intensity at the other detector position. On the basis thereof, the two UV radiation sources 21, 22, 23, 24 closest to the detector position where the notable change of the internal radiation intensity is detected may be identified as involved UV radiation sources 21, 22, 23, 24 whereas the other UV radiation sources 21, 22, 23, 24 are not. By not only taking into account the extent to which a change of the internal radiation intensity occurs at each of the detector positions, but also combining information from the detector positions, additional indications which help in identifying UV radiation sources 21, 22, 23, 24 as involved UV radiation sources 21, 22, 23, 24 are obtained, which is beneficial to accuracy of the identification procedure. The option of quickly "scanning" each of the UV radiation sources 21, 22 by putting each of the UV radiation sources 21, 22 to the normal state for a short period of time, as referred to in the foregoing, is still applicable here.

Fig. 4 diagrammatically shows an object 100 that can be equipped with one or more systems according to the invention, as an example of the many objects included in the scope of protection of the invention. The object 100 is especially suitable to be used in a public transport vehicle such as a bus, streetcar or a train, for example, and comprises two support poles 101, a handgrip rail 102 extending between the support poles 101, and handgrip elements 103 hanging down from the handgrip rail 102. Hence, the object 100 is intended to provide support to people traveling on the public transport vehicle. People may take hold of the object 100 by gripping a support pole 101, the handgrip rail 102 or a handgrip element 103 with one or two hands and/or may lean against a support pole 101. In order to prevent spread of diseases through the object 100, it is advantageous if systems according to the invention are applied to realize disinfection of the exterior of the object 100 at the position of all of the support poles 101, the handgrip rail 102 and the handgrip elements 103. Further, as the object 100 can be expected to be regularly touched by human beings, the capability of the systems according to the invention to automatically cause interruption of the default disinfecting functionality at the very position of touch and to thereby avoid harmful exposure of the human beings to the UV radiation 25 used for having the disinfecting functionality is most practical. The object 100 can be of conventional design, in which case the systems according to the invention or at least the radiation body 10 of the systems and components arranged in the radiation body 10 can be arranged on an exterior surface of the object 100, or the object 100 can be of adapted design.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention are summarized as follows. In a system 1, 2, 3 comprising a radiation body 10, a radiation arrangement 20 configured to provide radiation at least comprising UV radiation 25 to the radiation body 10, a detector arrangement 30 configured to detect an internal radiation intensity of radiation in the radiation body 10 at at least one detector position, and a controller arrangement 40 that is functionally coupled to the radiation arrangement 20 and the detector arrangement 30, the controller arrangement 40 is configured to apply a controller algorithm designed to set a state of UV radiation sources 21, 22, 23, 24 of the radiation arrangement 20 in dependence on input from the detector arrangement 30. In particular, when the input from the detector arrangement 30 represents a change of the internal radiation intensity that is qualified as a disturbance incident at the at least one detector position, an action of identifying at least one involved UV radiation source 21, 22, 23, 24 of which a change of contribution to an internal radiation intensity of UV radiation is demonstrated through occurrence of the disturbance incident and an action of setting an adapted state of the at least one involved UV radiation source 21, 22, 23, 24 are performed. In that way, it is possible to realize a functionality of the system 1, 2, 3 on the basis of which it is achieved that in a situation of an object 5 that is considerably larger than a microorganism touching a radiation exit window 11 of the radiation body 10, the extent to which the object 5 is exposed to the UV radiation 25 is automatically put to a safe level while normal, default operation of the UV radiation sources 21, 22, 23, 24 which are not identified as involved UV radiation sources 21, 22, 23, 24 is continued so that the disinfecting functionality of the radiation arrangement 20 is compromised to a minimum extent only.

## Claims

1. A system (1, 2, 3) comprising:
- a radiation body (10) comprising a radiation exit window (11), wherein the radiation body (10) is configured to receive radiation that at least comprises UV radiation (25), and to radiate at least part of the radiation to the exterior of the radiation body (10) via the radiation exit window (11);
- a radiation arrangement (20) configured to provide the radiation, comprising at least two UV radiation sources (21, 22, 23, 24) configured to emit UV radiation (25);
- a detector arrangement (30) configured to detect an internal radiation intensity of radiation in the radiation body (10) at at least one detector position; and
- a controller arrangement (40), functionally coupled to the radiation arrangement (20) and the detector arrangement (30), configured to set a normal state of the UV radiation sources (21, 22, 23, 24) as a default, in which state the UV radiation sources (21, 22, 23, 24) provide the UV radiation (25) with an intensity at an operational level, and configured to receive input from the detector arrangement (30) and to apply a controller algorithm that involves i) determining at least one safety test value that is representative of a parameter of the internal radiation intensity detected by the detector arrangement (30) at the at least one detector position, ii) assessing whether the at least one safety test value is inside or outside of a safety range of values associated with the at least one detector position, iii) after occurrence of a disturbance incident in which the at least one safety test value shifts from inside the safety reference range of values to outside of the safety reference range of values, identifying at least one involved UV radiation source (21, 22, 23, 24) of which a change of contribution to an internal radiation intensity of UV radiation (25) is demonstrated through occurrence of the disturbance incident, and iv) setting an adapted state of the at least one involved UV radiation source (21, 22, 23, 24), in which state the at least one involved UV radiation source (21, 22, 23, 24) provides the UV radiation (25) with an intensity at a level that is reduced relative to the operational level.

2. The system (1, 2, 3) according to claim 1, wherein the controller algorithm involves identifying the at least one involved UV radiation source (21, 22, 23, 24) on the basis of a predetermined relation between the UV radiation sources (21, 22, 23, 24) and the at least one detector position.

3. The system (1, 2, 3) according to claim 1, wherein the controller algorithm involves identifying the at least one involved UV radiation source (21, 22, 23, 24) by performing an identification procedure that includes i) controlling the radiation arrangement (20) to realize different UV radiation situations by successively putting different radiation subsets including at least one UV radiation source (21, 22, 23, 24) to the normal state while keeping all of the other UV radiation subsets in an inactive state, ii) controlling the detector arrangement (30) to detect the internal radiation intensity of the UV radiation (25) at the at least one detector position in respect of each of the different UV radiation situations, iii) determining an identification test value that is representative of a value of the internal radiation intensity detected by the detector arrangement (30) at the at least one detector position in respect of each of the different UV radiation situations, and iv) assessing whether the identification test value is inside or outside of an identification range of values associated with the at least one detector position in respect of each of the different UV radiation situations.

4. The system (1, 2, 3) according to any of claims 1-3, wherein the at least one safety test value is representative of a value of the internal radiation intensity of the radiation detected by the detector arrangement (30).

5. The system (1, 2, 3) according to claim 4, wherein the controller arrangement (30) is configured to apply a failure detection algorithm that involves assessing whether or not a time trend of the safety test value in periods of default operation of the UV radiation sources (21, 22, 23, 24) shows a structural change.

6. The system (1, 2, 3) according to any of claims 1-5, wherein the at least one safety test value is representative of a value of change in predetermined direction of the internal radiation intensity of the radiation detected by the detector arrangement (30).

7. The system (1, 2, 3) according to any of claims 1-6, wherein the reduced level of the intensity of the UV radiation (25) provided by the at least one involved UV radiation source (21, 22, 23, 24) in the adapted state is a zero level.

8. The system (1, 2, 3) according to any of claims 1-7, wherein the controller arrangement (40) is configured to enable adjustment of the safety reference range of values associated with the at least one detector position.

9. The system (1, 2, 3) according to claim 8, wherein the controller arrangement (40) is configured to adjust the safety reference range of values on the basis of a preceding value of the internal radiation intensity detected at the at least one detector position during a period directly prior to occurrence of a disturbance incident so as to include the preceding value and a predetermined tolerance window.

10. The system (1, 2, 3) according to claim 8, wherein the controller arrangement (40) is configured to apply a calibration algorithm that involves determining a calibration value of the internal radiation intensity detected at the at least one detector position during calibration periods at predetermined calibration intervals, and that involves adjusting the safety reference range of values on the basis of the calibration value of the internal radiation intensity so as to include the calibration value and a predetermined tolerance window.

11. The system (1, 2, 3) according to any of claims 1-10, wherein the controller algorithm involves maintaining the normal state of the at least one involved UV radiation source (21, 22, 23, 24) during a delay period of predetermined duration directly after occurrence of a disturbance incident, and setting the adapted state of the at least one involved UV radiation source (21, 22, 23, 24) directly after expiry of the delay period only when there is no occurrence during the delay period of a restoration incident involving a reversed shift of the at least one safety test value.

12. The system (1, 2, 3) according to any of claims 1-11, wherein the radiation body (10) comprises a slab of material, and wherein the UV radiation sources (21, 22, 23, 24) of the radiation arrangement (20) are embedded in the slab of material.

13. The system (1, 2, 3) according to any of claims 1-12, comprising at least one radiation source configured to provide radiation of distinctly longer wavelengths than UV wavelengths, wherein the detector arrangement (30) is configured to at least detect the internal radiation intensity of the radiation of the distinctly longer wavelengths.

14. An object (100) comprising the system (1, 2, 3) according to any of claims 1-13, wherein the radiation exit window (11) of the radiation body (10) of the system (1, 2, 3) is arranged to be at an exterior side of the object (100).

15. Method of adding a system (1, 2, 3) according to any of claims 1-13 to an existing object (100), wherein the radiation body (10) of the system (1, 2, 3) is applied to an exterior surface of the existing object (100).
